# FASCICULE DE BREVET EUROPEEN

(11) **EP 2 234 921 B1**
(45) Date de publication et mention de la délivrance du brevet: **13.03.2013**
(21) Numéro de dépôt: 09704303.8
(22) Date de dépôt: 12.01.2009
(51) Int. Cl.: C07C 253/10, C07C 255/04, C01C 3/02, C07C 4/06, C07C 9/04

(54) **PROCEDE DE FABRICATION D´ADIPONITRILE PAR HYDROCYANATION DU BUTADIÈNE**
VERFAHREN ZUR HERSTELLUNG VON ADIPONITRIL DURCH HYDROCYANIERUNG VON BUTADIEN
METHOD OF PRODUCTION OF ADIPONITRILE BY HYDROCYANATION OF BUTADIEN

(30) Priorité: 18.01.2008 FR 0800255
(43) Date de publication de la demande: 06.10.2010
(73) Titulaire: Rhodia Opérations, 93306 Aubervilliers (FR); Centre National de la Recherche Scientifique, 75794 Paris Cedex 16 (FR)
(72) Inventeur: MARION, Philippe, Vernaison 69390 (FR); HYNAUX, Amélie, F-78370 Plaisir (FR); LAURENTI, Dorothée, F-69140 Rillieux-la-pape (FR); GEANTET, Christophe, F-01700 Miribel (FR)
(74) Mandataire: Chatelan, Florence Anne
(86) Numéro de dépôt international: PCT/EP2009/050253
(87) Numéro de publication internationale: WO 2009/092637

(56) Documents cités:
- FR-A- 2 847 898
- FR-A- 2 857 965
- ANDRADE ET AL: "Catalytic Hydrogenolysis : III. Direct transformation of Cyano into methyl groups" SYNTHESIS,, 1 octobre 1980 (1980-10-01), pages 802-803, XP002493529
- BIANCHINI C ET AL: EUROPEAN JOURNAL OF INORGANIC CHEMISTRY,, 1 janvier 2001 (2001-01-01), pages 43-68, XP002493550
- F. ENDTER: "Die technische Synthese von Cyanwasserstoff aus Methan und Ammoniak ohne Zusatz von Sauerstoff." CHEMIE INGENIEUR TECHNIK, vol. 30, no. 5, 1958, pages 305-310, XP002496828

## Description

La présente invention concerne un procédé de fabrication d'adiponitrile par hydrocyanation du butadiène. Ce procédé comprend une étape de traitement des sous-produits non valorisables pour les transformer en ammoniac et composés hydrocarbonés valorisables.

Les procédés de fabrication d'adiponitrile sont produits industriellement à grande échelle. Parmi ces procédés, celui utilisant la réaction d'hydrocyanation du butadiène en deux étapes est exploité depuis les années 1970. La production mondiale d'adiponitrile est obtenue très majoritairement par ce procédé.

L'adiponitrile est un grand intermédiaire chimique utilisé dans la production d'hexaméthylène diamine, monomère important pour la fabrication de polymères, notamment de polyamides et pour celle d'isocyanates. Le procédé d'hydrocyanation du butadiène, consiste dans une première étape à additionner une molécule d'acide cyanhydrique sur une double liaison pour produire des composés insaturés mononitriles. Les composés mononitriles obtenus sont des composés linéaires comme le 2-pentènenitrile et le 3-pentenenitrile ou des composés ramifiés, comme le 2-méthyl 2-butène-nitrile, le 2-méthyl-3-butènenitrile.

Dans une seconde étape, une seconde molécule d'acide cyanhydrique est additionnée sur l'insaturation des composés mononitriles. Parmi les mononitriles cités précédemment, seul le 3-pentenenitrile peut être transformé en adiponitrile, les autres composés conduisant à des composés dinitriles ramifiés non valorisables pour la production d'hexaméthylène diamine.

De plus, l'addition de HCN sur l'insaturation du 3-pentènenitrile, permet d'obtenir majoritairement l'adiponitrile, mais également du 2-méthyl glutaronitrile (MGN) et du 2-éthylsuccinonitrile (ESN). La quantité de MGN produite est plus ou moins importante selon la nature du système catalytique utilisé. En outre, les composés mononitriles, autres que le 3-PN, introduits en seconde étape sont soit transformés en composés dinitriles différents de l'adiponitrile et non valorisables pour la production d'hexaméthylène diamine soit ne sont pas hydrocyanés. Ainsi, le composé 2-pentènenitrile (2-PN) n'est pas hydrocyané et est récupéré par séparation par distillation sous forme d'un flux de sous-produits non valorisables.

Pour améliorer la sélectivité de la première étape en composé 3-pentènenitrile, le procédé de fabrication d'adiponitrile comprend une étape d'isomérisation qui permet notamment de transformer le 2-méthyl-3-butènenitrile en 3-pentènenitrile.

Dans le présent texte, on entend par composés ou produits valorisables, les composés qui sont utilisables comme matières premières dans des réactions chimiques de synthèse de composés chimiques importants comme l'adiponitrile les diamines (hexaméthylène diamine), les aminonitriles (aminocapronitrile). Les composés nitriles valorisables sont choisis parmi l'adiponitrile, le 3-pentènenitrile, le 4-pentènenitrile, le 2-méthyl-3-butènenitrile ou leurs mélanges. Par sous-produits non valorisables, il faut comprendre, les produits formés au cours du procédé d'hydrocyanation qui ne peuvent être économiquement utilisés comme matière première pour la fabrication de nouveaux produits et qui sont considérés dans le procédé d'hydrocyanation comme des effluents à traiter avant leur rejet dans l'environnement. Les sous-produits non valorisables sont choisis parmi le méthylglutaronitrile, l'éthylsuccinonitrile, le 2-pentènenitrile, le 2-methyl-2-bebtènenitrile ou leurs mélanges.

Ces sous-produits non valorisables sont le plus souvent détruits par incinération dans des chaudières pour la production de vapeur. Toutefois, certains de ceux-ci peuvent être valorisés totalement ou partiellement par transformation chimique en de nouveaux composés utiles. Ainsi, le sous-produit le plus important en quantité est le 2-méthyl-glutaronitrile (MGN) qui peut être notamment hydrogéné pour produire une diamine ramifiée la 2-méthyl-pentaméthylène diamine (MPMD) utilisée principalement comme monomère pour la fabrication de polyamide ou comme matière première pour la synthèse de produits chimiques. D'autres valorisations du MGN ont été décrites.

Les autres sous-produits dinitriles ou mononitriles sont essentiellement valorisés par combustion pour produire de l'énergie. Toutefois, comme ces composés comprennent des atomes d'azote, les gaz de combustion produits contiennent des oxydes d'azote. Ainsi, il peut être nécessaire de traiter ces gaz de combustion dans des unités de transformation et destruction des oxydes d'azote appelées DENOx.

Le problème de traitement et de valorisation des sous-produits non valorisables dans le procédé d'hydrocyanation du butadiène n'est donc toujours pas entièrement résolu et de nouvelles solutions sont constamment recherchées.

Le document FR 2857965 décrit la production et la séparation de dinitrile par hydrocyanation de mononitriles insaturés suivie d'une distillation pour récupérer la fraction des dinitriles comme fraction intermédiaire.

Le document J. G Andrade et al., Synthesis 802-803 (1980) décrit la conversion de groupes cyano en groupes méthyle de composés nitriles aromatiques ou tertiaires.

Un des buts de la présente invention est de proposer un procédé de traitement de ces sous-produits ne présentant pas les inconvénients de la combustion et permettant d'améliorer l'économie globale du procédé, notamment en valorisant les sous-produits sous forme de composés valorisables et avantageusement recyclables dans le procédé de fabrication de l'adiponitrile.

A cet effet, l'invention propose un procédé de production d'adiponitrile par hydrocyanation du butadiène en présence d'un système catalytique comprenant une ou des étapes de séparation des composés nitriles valorisables et des sous-produits nitriles non valorisables du ou des milieux d'hydrocyanation, caractérisé en ce qu'il consiste à traiter les sous-produits non valorisables dans une étape d'hydrodésazotation ou hydrotraitement par réaction avec de l'hydrogène sous une pression absolue comprise entre 0,1 et 10 MPa, de préférence de 0,5 MPa à 3 MPa, à une température comprise entre 200 et 500°C, de préférence de 300°C à 400°C et en présence d'un catalyseur d'hydrodésazotation pour transformer ces sous-produits en ammoniac et composés hydrocarbonés.

Le procédé de l'invention permet de traiter la totalité ou une partie des flux de sous-produits non valorisables comprenant des fonctions nitriles générés dans le procédé d'hydrocyanation du butadiène pour récupérer l'atome d'azote sous forme ammoniac et la majeure partie des atomes de carbone et d'hydrogène sous forme de composés hydrocarbonés comprenant de 1 à plusieurs atomes de carbone.

Au cours de cet hydrotraitement, il peut se produire également un craquage thermique des chaines hydrocarbonés conduisant à la formation de composés hydrocarbonés sans atome d'azote et/ou de composés hydrocarbonés comprenant des atomes d'azote. Ces derniers pourront être transformés en composés hydrocarbonés par réaction avec l'hydrogène, selon les conditions opératoires mises en oeuvre. Par ailleurs, des composés cycliques contenant des atomes d'azote peuvent également se former comme les picolines. Selon l'invention, on appelle % HDN ou rendement du procédé de traitement de l'invention, le rapport exprimé en pourcentage, du nombre de moles de composés hydrocarbonés ne comprenant pas d'atome d'azote produites soit par hydrotraitement soit par craquage thermique par rapport au nombre de moles de composés à traiter engagées.

Après séparation et récupération de l'ammoniac, ces composés hydrocarbonés peuvent être valorisés tels quels ou alimentés dans une étape de vaporéformage et de méthanation pour être transformés en méthane, produit valorisable notamment comme générateur d'énergie et matière première pour la synthèse de nombreux composés comme notamment l'acide cyanhydrique, l'hydrogène.

Ainsi, dans le procédé d'hydrocyanation du butadiène, l'acide cyanhydrique (HCN), un des principaux réactifs du procédé, est généré en amont de l'étape d'hydrocyanation. Cette production d'acide cyanhydrique est généralement réalisée par mise en oeuvre du procédé ANDRUSSOW qui consiste à faire réagir du gaz naturel dont le constituant principal est le méthane, avec de l'ammoniac en présence d'oxygène ou d'un gaz contenant de l'oxygène.

Le procédé de l'invention, qui produit de l'ammoniac et du méthane par hydrodésazotation des sous-produits non valorisables et vaporéformage/méthanation des composés hydrocarbonés obtenus permet de recycler, avantageusement, l'ammoniac et le méthane ainsi produits dans l'étape de synthèse de l'acide cyanhydrique. De ce fait, les quantités d'ammoniac et de gaz naturel ou méthane consommées par tonne d'adiponitrile produite sont significativement réduites.

Selon une autre caractéristique de l'invention, le catalyseur d'hydrodésazotation comprend un élément métallique appartenant au groupe des métaux nobles constitué par le platine, le palladium, le rhodium, le ruthénium ou un métal de transition tel que le nickel. De préférence, on utilisera le platine.

Avantageusement et préférentiellement le catalyseur est du type catalyseur supporté dans lequel l'élément catalytique métallique est supporté sur un matériau, préférentiellement poreux, tels que l'alumine, la silice, les aluminosilicates, les silice-alumines, les charbons actifs, la zircone, l'oxyde de titane, les zéolithes.

Le catalyseur préféré de l'invention comprend du platine déposé sur un support choisi dans le groupe comprenant la silice, l'alumine, la zircone, les silice-alumines, les zéolithes.

La réaction d'hydrodésazotation ou hydrotraitement est réalisée en présence d'un catalyseur hétérogène qui est soit dispersé en suspension dans le réacteur soit sous forme de lit fixe ou lit fluidisé à travers lequel le flux de composés nitriles est alimenté. Le catalyseur peut être aussi déposé sur un support monolithique en forme de nid d'abeilles.

La présente invention n'est pas limitée à ces modes de réalisation donnés uniquement à titre d'illustration.
Les catalyseurs d'hydrodésazotation préférés de l'invention sont notamment les catalyseurs platine sur zircone, platine sur silice-alumine.

Le taux de transformation (% HDN) des composés nitriles engagés est très élevé, proche ou égal à 100%. Les produits récupérés sont l'ammoniac et majoritairement des composés hydrocarbonés. Ainsi, le traitement du méthyl-2-glutaronitrile permet d'obtenir comme composés hydrocarbonés, très majoritairement du méthyl-2-pentane.

Le mélange de composés récupérés est avantageusement traité pour séparer et récupérer l'ammoniac, par exemple par distillation.

Selon une caractéristique préférée de l'invention, les composés hydrocarbonés, notamment le méthyl-2 pentane, peuvent être soumis à un enchaînement comprenant un vaporéformage ou steamreforming suivi d'une méthanation pour produire du méthane. Ce traitement de vaporeformage/méthanation est très utilisé dans l'industrie pétrolière et peut être mis en oeuvre par chauffage en présence d'un catalyseur (vaporeformage catalytique). Comme catalyseurs usuels pour ces réactions, on peut citer les nickels supportés. Ce procédé est réalisé à une température comprise entre 200 et 700°C et sous une pression comprise entre 5 et 50 bar.

Il peut aussi être intéressant de soumettre les composés hydrocarbonés obtenus à l'étape d'hydrodésazotation uniquement à un traitement de vaporéformage. L'hydrogène et l'oxyde de carbone récupérés peuvent être directement valorisés ensemble ou séparément.

Quand les composés hydrocarbonés formés dans l'étape d'hydrodésazotation doivent être soumis à un vaporéformage, il est préférable d'éliminer les traces d'ammoniac contenu dans ces composés hydrocarbonés pour ne pas affecter les performances du vaporéformage et de la méthanation.

Une description générale des procédés de vaporéformage thermique ou catalytique et méthanation est donnée dans l'ouvrage « Les procédés de pétrochimie » Edition TECHNIP Tome 1 1965 dont les auteurs sont A. CHAUVEL, G. LEFEBVRE et L. CASTEX.

Le procédé de l'invention s'applique au procédé de fabrication d'adiponitrile par hydrocyanation du butadiène en deux étapes. Ce procédé est décrit dans de nombreux brevets et une description détaillée est disponible dans les RAPPORTS SRI n° 31 suppl B intitulé « HEXAMETHYLENE DIAMINE ».

Brièvement, dans un procédé d'hydrocyanation du butadiène, la première étape consiste à fixer une molécule de HCN sur une double liaison du butadiène. Cette étape est réalisée en présence d'un système catalytique comprenant un élément métallique, préférentiellement le nickel, complexé avec des ligands organophosphorés comprenant un ou plusieurs atomes de phosphore. Ces ligands organophosphorés sont choisis parmi les organophosphites, organophosphonites, organophosphinites et organophosphines. Parmi ces ligands, le tritolylphosphite est utilisé industriellement depuis les années 1970.

D'autres composés comprenant deux atomes de phosphore appelés ligands bidentates ont aussi été proposés dans de nombreux brevets. Il a également été proposé d'utiliser un système catalytique comprenant un mélange de ligands monodentate organophosphite et un ligand bidentate permettant d'utiliser une quantité diminuée de ligand bidentate par rapport au nickel.

Les composés mononitriles formés à cette étape sont nombreux. On peut citer comme composés formés les mononitriles linéaires 2-pentenenitrile (2PN) et 3-pentènitrile (3PN), les mononitriles ramifiés tels que 2-méthyl-2-butènenitrile (2M2BN) et le 2-méthyl-3-butènenitrile (2M3BN).

Comme indiquée précédemment, une étape d'isomérisation est prévue pour convertir le maximum de ces composés mononitriles ramifiés (principalement le 2M3BN) en composé 3-pentenitrile, seul précurseur d'adiponitrile.

La première étape génère un flux de sous-produits non valorisables qui n'est pas alimenté en deuxième étape, contenant principalement du 2M2BN.

Dans une seconde étape décrite précédemment, le système catalytique peut être équivalent ou similaire à celui de la première étape mais un cocatalyseur tel qu'un acide de Lewis est ajouté à ce système catalytique. Dans cette étape, le 3-pentènenitrile est transformé en adiponitrile mais également en d'autres dinitriles ramifiés (méthylglutaronitrile, éthylsuccinonitrile) qui sont séparés pour former un flux d'effluents ou de sous-produits non valorisables en hexaméthylène diamine. Dans cette deuxième étape, la séparation des différents produits contenus dans le milieu réactionnel permet de récupérer le 2-pentènenitrile alimenté depuis la première étape et qui n'a pas été transformé en composés dinitriles. Ce flux de 2-pentènenitrile est également un flux de sous-produits non valorisables qui sera traité selon le procédé de l'invention.

Selon le procédé de l'invention, les flux d'effluents ou sous-produits nitriles non valorisables en 3-PN ou hexaméthylène diamine récupérés en 1^{ère} et 2^{ème} étapes peuvent être rassemblés et traités pour être décomposés en ammoniac et alcanes, ou un seul de ces flux peut être traité. Il peut être intéressant de traiter ces flux d'effluents avant de les soumettre à l'étape d'hydrodésazotation pour, par exemple, éliminer certains composés tels que les composés phosphorés qui pourraient être des poisons pour les catalyseurs d'hydrodésazotation et/ou de vaporéformage et/ou de méthanation.

Comme indiqué précédemment, l'ammoniac et le méthane obtenus par le procédé de l'invention sont avantageusement recyclés dans l'installation de synthèse de HCN généralement associée à une installation de production d'adiponitrile.

D'autres avantages, détails de l'invention apparaîtront plus clairement au vu des exemples qui seront donnés ci-après à titre d'illustration uniquement.

Les essais décrits ci-après ont été réalisés avec deux catalyseurs d'hydrodésazotation :
- Catalyseur A : Pt déposé sur de la zircone (Pt/ZrO₂)
- Catalyseur B : Platine déposé sur une silice-alumine comprenant un un pourcentage en poids de silice égal à 10, appelé Pt/SiAl10.

Le catalyseur A a été obtenu en utilisant un support zircone de surface spécifique égale à 83 m²/g.

Le catalyseur B comprend un support silice-alumine de surface spécifique égale à 352 m²/g commercialisée par la société Condéa sous l'appellation commerciale SIRAL10. Ce support contient 10 % en poids de SiO₂.

Ces catalyseurs sont préparés selon le mode opératoire ci-dessous:

Les supports sont imprégnés par une solution d'acide hexachloroplatinique H₂PtCl₆. Ils sont laissés à maturation pendant deux heures à température ambiante, afin de permettre à la solution de pénétrer dans les pores. Les produits sont ensuite séchés pendant une nuit (> 12 h) à 110 °C puis calcinés sous flux d'air à 500°C pendant 1 heure (débit d'air de 60 cm³.min⁻¹, rampe de montée en température de 2°C.min⁻¹), afin de décomposer le complexe précurseur en oxyde de platine. Ils sont ensuite réduits sous un flux d'hydrogène pendant 6 heures à 310°C (débit d'hydrogène de 60 cm³.min⁻¹, rampe de montée en température de 1°C.min⁻¹) pour obtenir un dépôt de platine métallique.

Les caractéristiques physico-chimiques des catalyseurs Pt/ZrO₂ et Pt/SiAl10 sont rassemblées dans le tableau I.

La dispersion et la taille des particules de platine ont été déterminées par chimisorption d'hydrogène. Le dosage du platine a été effectué par spectrométrie d'émission plasma.

**Tableau I**

| Catalyseur | % massique Pt | dispersion [%] | t_{particule} [nm] |
|---|---|---|---|
| A | 1,1 | 60 | 1,7 |
| B | 1,1 | 66 | 1,4 |

Dans les exemples 1 à 4, les abréviations utilisées ont les significations indiquées ci-dessous :
MP : 2-méthylpentane
MPip : 3-méthylpiperidine
Pic : picolines (β-picoline, 2-amino-3-picoline, 6-amino-3-picoline)
% HDN : pourcentage de produits hydrocarbonés ne contenant pas d'atome d'azote par rapport au nombre de moles de composés à traiter engagés

Exemple 1 : Hydrodésazotation du MGN sous une pression absolue de 0,1 MPa avec le catalyseur A

La réaction d'hydrodésazotation (HDN) du méthylglutaronitrile a été réalisée à différentes températures et sous une pression absolue de 0,1 MPa avec un débit d'hydrogène de 55 ml/min et un lit fixe de catalyseur A de masse 15 mg, selon le mode opératoire suivant dans un microréacteur dynamique.

Le mélange réactionnel comprend du 2-méthylglutaronitrile pur et de l'hydrogène. L'hydrogène, dont le débit est régulé par un débitmètre massique (0 - 200 ml/min), barbote dans un saturateur rempli de MGN liquide, puis passe dans un condenseur dont la température contrôle la pression partielle du MGN. La pression partielle de MGN est de 1,33 kPa. Le réacteur est placé dans un four tubulaire dont la température est contrôlée par un régulateur à sonde de platine. La température de réaction est mesurée grâce à un thermocouple situé au niveau du lit catalytique.

Afin d'éviter la condensation du réactif et des produits de réaction, la température de l'ensemble de l'appareil est constamment maintenue à 180 °C. Un piège est situé en sortie du test pour condenser les produits de réaction et le réactif non converti. Les gaz partent ensuite à l'évent. Les composés récupérés sont principalement comme composés hydrocarbonés le 2-méthylpentane (MP) et comme composés comprenant un atome d'azote la 3-méthylpipéridine (MPip) et la picoline ou ses dérivés désignés globalement par Pic.

Les différents rendements obtenus sont rassemblés dans le tableau II ci-dessous :

**Tableau II**

| T [°C] | 250 | 300 | 350 | 400 | 450 |
|---|---|---|---|---|---|
| Produits azotés (dont Pic et MPip) [%] | 70,3 (3,6-61,1) | 78,6 (65-5,3) | 74 (57,6-0) | 64,9 (27,7-0) | 67 (10,9-0) |
| Produits hydrocarbonés (dont MP) [%] | 0,3 (0,3) | 2,6 (1,7) | 3,7 (1,2) | 13 (0,7) | 12 (0,2) |

Exemple 2 : Hydrodésazotation du MGN sous une pression absolue de 0,1 MPa avec le catalyseur B

L'exemple 1 est répété à l'exception du type de catalyseur qui est le catalyseur B.

Les rendements obtenus sont rassemblés dans le tableau III ci-dessous :

**Tableau III**

| T [°C] | 250 | 300 | 350 | 400 | 450 |
|---|---|---|---|---|---|
| Produits azotés (dont Pic et MPip) [%] | 61,3 (4,4-50) | 68,3 (57,5-2,5) | 65,7 (48-0) | 58,7 (25,9-0) | 43,8 (9,9-0) |
| Produits hydrocarbonés (dont MP) [%] | 0,3 (0,3) | 1,4 (1,1) | 4,8 (1,4) | 18,3 (1,2) | 40,4 (0,7) |

Exemple 3 : Hydrodésazotation du MGN sous une pression absolue de 1 MPa avec le catalyseur A

L'exemple 1 est répété en utilisant 50 mg de catalyseur A avec une pression absolue de 1 MPa, une pression partielle de MGN égale à 1,33kPa et un débit d'hydrogène de 16 ml/min. Quand les essais sont effectués sous pression, le mélange réactionnel est injecté après détente à la pression atmosphérique dans un chromatographe en phase gaz par l'intermédiaire d'une vanne six voies.

Les rendements obtenus sont rassemblés dans le tableau IV ci-dessous :

**Tableau IV**

| T [°C] | 250 | 300 | 350 | 400 |
|---|---|---|---|---|
| Produits azotés (dont Pic et MPip) | 93,9 (1,5-92,4) | 67,1 (15,4-51,7) | 46,1 (41,8-4,3) | 55,9 (55,6) |
| [%] | | | | |
| Produits hydrocarbonés (dont MP) [%] | 4,1 (2,8) | 32,1 (32,1) | 49,5 (44) | 22,2 (11,4) |

Exemple 4 : Hydrodésazotation du MGN sous une pression absolue de 1 MPa avec le catalyseur B

L'exemple 3 est répété à l'exception du type de catalyseur qui est le catalyseur B.

Les rendements obtenus sont rassemblés dans le tableau V ci-dessous

**Tableau V**

| T [°C] | 250 | 300 | 350 | 400 |
|---|---|---|---|---|
| Produits azotés (dont Pic et MPip) [%] | 65,6 (0,9-64,7) | 4,4 (1,3-3,1) | 0 (48) | 3,4 (3,4-0) |
| Produits hydrocarbonés (dont MP) [%] | 34,4 (32,9) | 95,6 (90,7) | 100 (86,3) | 96,6 (54,9) |

Exemple 5 : Hydrodésazotation du MGN sous une pression absolue de 0,55 MPa avec le catalyseur B

L'exemple 4 est répété sous une pression absolue de 0,55 MPa, un débit d'hydrogène de 4 ml/min et une pression partielle de MGN égale à 1,33 kPa.

Les rendements obtenus sont rassemblés dans le tableau VI ci-dessous

**Tableau VI**

| T [°C] | 250 | 300 | 350 |
|---|---|---|---|
| Produits azotés (dont Pic et MPip) [%] | 30,3 (2,5-27,8) | 0 (0-0) | 0,3 (0,3-0) |
| Produits | 69,8 | 100 | 99,7 |
| hydrocarbonés (dont MP) [%] | (68,6) | (93,9) | (78,5) |

Ces résultats montrent que l'activité catalytique en hydrodésazotation du MGN est faible sous une pression atmosphérique pour une température comprise entre 250°C < et < 350°C.

Sous une pression de 1 MPa, le rendement de l'hydrodésazotation du MGN est plus élevé et atteint une valeur de 100 % pour une température de 350°C.

Sous une pression de 0,55 MPa, il est également possible d'obtenir un rendement de l'hydrodésazotation du MGN de 100 % pour une température de 300°C.

Exemple 6 : Hydrodésazotation d'un sous-produit issu d'une unité de fabrication d'adiponitrile par hydrocyanation de butadiène, sur le catalyseur B.

Les producteurs d'adiponitrile commercialisent certains sous-produits ou effluents provenant de la fabrication de l'adiponitrile par hydrocyanation du butadiène. Ainsi, le sous-produit contenant principalement du méthyl-2-glutaronitrile représentant une fraction de distillation récupérée dans le procédé de séparation de l'adiponitrile est commercialisé sous l'appellation flux MGN.

Ce flux MGN a été utilisé pour réaliser les exemples 6 et suivants. Il a la composition pondérale suivante :
● 2-méthylglutaronitrile : 85 %
● Ethylsuccinonitrile : 11 %
● Adiponitrile : 3 %
● Autres composés : 1 %
La réaction d'hydrodésazotation (HDN) du MGN brut ci-dessus a été réalisée à différentes températures, sous une pression absolue de 0.55 MPa (débit d'hydrogène de 4 ml/min et pression partielle de MGN de 1,33kPa) et une masse de catalyseur B de 50 mg, selon le mode opératoire décrit à l'exemple 5.

**Tableau VII**

| T [°C] | 300 | 350 | 400 |
|---|---|---|---|
| Produits azotés en % | 4,1 | 3,5 | 9,3 |
| Produits hydrocarbonés en % | 92,1 | 93,3 | 88,3 |

Exemple 7 : Hydrodésazotation d'un sous-produit issu d'une unité de fabrication d'adiponitrile par hydrocyanation de butadiène :
L'exemple 6 a été répété sous une pression absolue de 1MPa, pour un débit d'hydrogène de 16 ml/min et une pression partielle en MGN de 1,33kPa. Dans ces conditions à 300°C, le %HDN est égal à 100%

Exemple 8

L'exemple 6 a été répété sous une pression absolue de 0,55 MPa mais en utilisant comme catalyseur, un catalyseur commercial platine supporté sur zéolithe commercialisé par la société Sud Chemie, Ce catalyseur contient 0,3 % en poids de Pt et a un rapport atomique Si/Al égal à 11,5.

Les résultats obtenus sont rassemblés dans le tableau VIII ci-dessous :

**Tableau VIII**

| T [°C] | 250 | 300 | 350 |
|---|---|---|---|
| Produits azotés [%] | 80,1 | 70,2 | 72,9 |
| Produits hydrocarbonés (%) | 2,9 | 22,1 | 26,1 |

Exemple 9 :

L'exemple 6 a été répété sous une pression absolue de 0,55 MPa mais en utilisant comme catalyseur, un catalyseur commercial platine supporté sur zircone commercialisé par la société Johnson Matthey. Ce catalyseur contient 1 % poids de Pt).

Les résultats obtenus sont rassemblés dans le tableau IX ci-dessous:

**Tableau IX**

| T [°C] | 250 | 300 | 350 |
|---|---|---|---|
| Produits azotés [%] | 65,4 | 2,6 | 4,7 |
| Produits hydrocarbonés (%) | 28,5 | 97,5 | 95,3 |

Exemple 10 : vaporéformage et méthanation du méthylpentane

Un flux de 5g/h de methylpentane est alimenté à un réacteur en phase gaz en parallèle à un flux d'eau de 7,5g/h.

Le réacteur contient environ 100 ml de catalyseur base nickel supporté sur alumine (70% poids de nickel).La température est maintenue vers 550°C par chauffage externe. La pression est régulée à 23 bar. En sortie le gaz est refroidi et alimenté directement dans un second réacteur contenant 100ml des catalyseur Ni supporté sur alumine opérant sous 16 bar et à 270°C. Ce second réacteur est aussi alimenté par un débit constant fixé à 15 Nl/h d'hydrogène. En sortie les gaz sont analysés par chromatographie en phase gaz.

La conversion du méthylpentane est supérieure à 98%. On ne détecte que le méthane comme produit hydrocarboné.

## Revendications

1. Procédé de production d'adiponitrile par hydrocyanation du butadiène en présence d'un système catalytique comprenant une ou des étapes de séparation du ou des milieux d'hydrocyanation, des composés nitriles valorisables choisis parmi l'adiponitrile, le 3-pentènenitrile, le 4-pentènenitrile, le 2-méthyl-3-butènenitrile ou leurs mélanges et des sous-produits nitriles non valorisables choisis parmi le méthylglutaronitrile, l'éthylsuccinonitrile, le 2-pentènenitrile, le 2-méthyl-2-butènenitrile ou leurs mélanges, **caractérisé en ce qu'**il consiste à traiter les sous-produits nitriles non valorisables dans une étape d'hydrodésazotation par réaction avec de l'hydrogène sous une pression absolue d'hydrogène comprise entre 0,1 et 10 MPa, une température comprise entre 200°C et 500°C en présence d'un catalyseur d'hydrodésazotation pour transformer lesdits sous-produits en ammoniac et composés hydrocarbonés.

2. Procédé selon la revendication 1, **caractérisé en ce que** le catalyseur d'hydrodésazotation est un élément métallique choisi dans le groupe comprenant le platine, le palladium, le rhodium, le ruthénium, le nickel.

3. Procédé selon la revendication 2, **caractérisé en ce que** le catalyseur comprend un élément métallique supporté sur un support choisi dans le groupe comprenant, l'alumine, la silice, les aluminosilicates, les silice-alumines, les charbons actifs, la zircone, l'oxyde de titane, les zéolithes.

4. Procédé selon la revendication 3, **caractérisé en ce que** le catalyseur comprend du platine déposé sur un support choisi dans le groupe comprenant la zircone, la silice, l'alumine, les aluminosilicates, les silice-alumines.

5. Procédé selon l'une des revendications précédentes, **caractérisé en ce que** la pression absolue en hydrogène est comprise entre 0.5 MPa et 3 MPa.

6. Procédé selon l'une des revendications précédentes, **caractérisé en ce que** la température est comprise entre 300°C et 400°C

7. Procédé selon l'une des revendications précédentes, **caractérisé en ce que** les composés hydrocarbonés récupérés à l'issue de l'étape d'hydrodésazotation sont traités dans une étape de vaporéformage et méthanation pour produire des alcanes inférieurs tels que le méthane, après séparation de l'ammoniac.

8. Procédé selon la revendication 7, **caractérisé en ce que** l'étape de vaporéformage et méthanation est mise en oeuvre en présence d'un catalyseur à base de nickel supporté à une température comprise entre 200 et 700°C et sous une pression comprise entre 5 et 50 bar.

9. Procédé selon l'une des revendications précédentes, **caractérisé en ce qu'**il comprend une étape de production d'acide cyanhydrique par réaction entre l'ammoniac et le méthane et **en ce que**, l'ammoniac formé à l'étape d'hydrodésazotation et/ou le méthane formé à l'étape de vaporeformage/méthanation sont alimentés dans l'étape de production de l'acide cyanhydrique.

## Claims

1. Process for the production of adiponitrile by hydrocyanation of butadiene in the presence of a catalytic system comprising one or more stages of separation of the hydrocyanation medium or media, of the nitrile compounds of economic value chosen from adiponitrile, 3-pentenenitrile, 4-pentenenitrile, 2-methyl-3-butenenitrile or their mixtures and nitrile by-products not of economic value chosen from methylglutaronitrile, ethylsuccinonitrile, 2-pentenenitrile, 2-methyl-2-butenenitrile or their mixtures, **characterized in that** it consists in treating the nitrile by-products not of economic value in a hydrodenitrogenation stage by reaction with hydrogen under an absolute hydrogen pressure of between 0.1 and 10 MPa, at a temperature of between 200°C and 500°C, in the presence of a hydrodenitrogenation catalyst, in order to convert the said by-products into ammonia and hydrocarbon compounds.

2. Process according to Claim 1, **characterized in that** the hydrodenitrogenation catalyst is a metal element chosen from the group consisting of platinum, palladium, rhodium, ruthenium and nickel.

3. Process according to Claim 2, **characterized in that** the catalyst comprises a metal element supported on a support chosen from the group consisting of alumina, silica, aluminosilicates, silicas/aluminas, active charcoals, zirconia; titanium oxide and zeolites.

4. Process according to Claim 3, **characterized in that** the catalyst comprises platinum deposited on a support chosen from the group consisting of zirconia, silica, alumina, aluminosilicates and silicas/aluminas.

5. Process according to one of the preceding claims, **characterized in that** the absolute hydrogen pressure is between 0.5 MPa and 3 MPa.

6. Process according to one of the preceding claims, **characterized in that** the temperature is between 300°C and 400°C.

7. Process according to one of the preceding claims, **characterized in that** the hydrocarbon compounds recovered on conclusion of the hydrodenitrogenation stage are treated in a steam reforming and methanation stage in order to produce lower alkanes, such as methane, after separation of the ammonia.

8. Process according to Claim 7, **characterized in that** the steam reforming and methanation stage is carried out in the presence of a supported nickel-based catalyst at a temperature of between 200°C and 700°C and under a pressure of between 5 and 50 bar.

9. Process according to one of the preceding claims, **characterized in that** it comprises a stage of production of hydrocyanic acid by reaction between ammonia and methane and **in that** the ammonia formed in the hydrodenitrogenation stage and/or the methane formed in the steam reforming/methanation stage are fed to the stage of production of hydrocyanic acid.

## Patentansprüche

1. Verfahren zur Herstellung von Adiponitril durch Hydrocyanierung von Butadien in Gegenwart eines Katalysatorsystems mit einem oder mehreren Schritten der Trennung des oder der Hydrocyanierungsmedien, aus Adiponitril, 3-Pentennitril, 4-Pentennitril, 2-Methyl-3-butennitril oder Gemischen davon ausgewählter verwertbarer Nitrilverbindungen und aus Methylglutarnitril, Ethylsuccinnitril, 2-Pentennitril, 2-Methyl-2-butennitril oder Gemischen davon ausgewählter nicht verwertbarer Nitrilnebenprodukte, **dadurch gekennzeichnet, dass** es darin besteht, dass man die nicht verwertbaren Nitrilnebenprodukte in einem Hydrodenitrogenierungsschritt durch Reaktion mit Wasserstoff unter einem Wasserstoff-Absolutdruck zwischen 0,1 und 10 MPa bei einer Temperatur zwischen 200°C und 500°C in Gegenwart eines Hydrodenitrogenierungskatalysators behandelt, wodurch die Nebenprodukte in Ammoniak und Kohlenwasserstoffverbindungen umgewandelt werden.

2. Verfahren nach Anspruch 1, **dadurch gekennzeichnet, dass** es sich bei dem Hydrodenitrogenierungskatalysator um ein Metallelement aus der Gruppe bestehend aus Platin, Palladium, Rhodium, Ruthenium und Nickel handelt.

3. Verfahren nach Anspruch 2, **dadurch gekennzeichnet, dass** der Katalysator ein auf einem Träger aus der Gruppe bestehend aus Aluminiumoxid, Siliciumoxid, Aluminosilikaten, Siliciumoxid-Aluminiumoxiden, Aktivkohlen, Zirkoniumoxid, Titanoxid und Zeolithen geträgertes Metallelement umfasst.

4. Verfahren nach Anspruch 3, **dadurch gekennzeichnet, dass** der Katalysator auf einem Träger aus der Gruppe bestehend aus Zirkoniumoxid, Siliciumoxid, Aluminiumoxid, Aluminosilikaten und Siliciumoxid-Aluminiumoxiden abgeschiedenes Platin umfasst.

5. Verfahren nach einem der vorhergehenden Ansprüche, **dadurch gekennzeichnet, dass** der Wasserstoff-Absolutdruck zwischen 0,5 MPa und 3 MPa liegt.

6. Verfahren nach einem der vorhergehenden Ansprüche, **dadurch gekennzeichnet, dass** die Temperatur zwischen 300°C und 400°C liegt.

7. Verfahren nach einem der vorhergehenden Ansprüche, **dadurch gekennzeichnet, dass** man die am Ende des Hydrodenitrogenierungsschritts gewonnenen Kohlenwasserstoffverbindungen nach Abtrennung des Ammoniaks in einem Dampfreformierungs- und Methanierungsschritt zur Herstellung von niederen Alkanen wie Methan behandelt.

8. Verfahren nach Anspruch 7, **dadurch gekennzeichnet, dass** man den Dampfreformierungs- und Methanierungsschritt in Gegenwart eines Katalysators auf der Basis von geträgertem Nickel bei einer Temperatur zwischen 200 und 700°C und unter einem Druck zwischen 5 und 50 bar durchführt.

9. Verfahren nach einem der vorhergehenden Ansprüche, **dadurch gekennzeichnet, dass** es einen Schritt der Herstellung von Cyanwasserstoffsäure durch Reaktion zwischen Ammoniak und Methan umfasst und man dem Schritt der Herstellung von Cyanwasserstoffsäure das bei dem Hydrodenitrogenierungsschritt gebildete Ammoniak oder das bei dem Dampfreformierungs-/Methanierungsschritt gebildete Methan zuführt.
